# EUROPEAN PATENT SPECIFICATION

(11) **EP 2 517 630 B1**
(45) Date of publication and mention of the grant of the patent: **14.05.2014**
(21) Application number: 12165024.6
(22) Date of filing: 20.04.2012
(51) Int. Cl.: A61B 8/12, A61M 25/10, A61M 25/00, A61J 15/00, A61M 25/01, A61B 19/00

(54) **Feeding tube having echogenic tip**
Ernährungssonde mit echogener Spitze
Tube d'alimentation ayant d'une pointe échogénique

(30) Priority: 26.04.2011 US 201113094270
(43) Date of publication of application: 31.10.2012
(73) Proprietor: Cook Medical Technologies LLC, Bloomington, IN 47402 (US)
(72) Inventor: Bosel, Christopher D., Bloomington, IN Indiana 47404 (US); Melsheimer, Jeffry S., Springville, IN Indiana 47462 (US); Irwin, Nathaniel A., Bloomington, IN Indiana 47401 (US); Kamel, Amro, Bloomington, IN Indiana 47403 (US)
(74) Representative: Garratt, Peter Douglas

(56) References cited:
- EP-A1- 2 478 929
- US-A- 5 967 988
- US-A1- 2008 200 874
- US-A1- 2008 300 571

## Description

### BACKGROUND OF THE INVENTION

Technical Field. The present invention relates to a tubular member having a lumen therethrough for transport of fluid material. More particularly, the invention relates to a feeding tube for insertion into the jejunum of a patient, wherein the feeding tube has an echogenically enhanced portion that permits location of the tube under ultrasound imaging.

Background Information. Patients for whom normal ingestion of food becomes difficult or impossible may require placement of a feeding tube to assist in providing their nutritional needs. For some individuals, such as comatose patients, stroke victims, or those with a compromised gastrointestinal tract, this may require placement of a tube that is introduced percutaneously into the stomach for delivery of nutritional products directly into the stomach. Such tubes for delivery of nutritional products into the stomach are generally referred to as gastrostomy tubes, or "G"-tubes.

In some situations, feeding a patient through a G-tube can be problematic. Such situations include, among others, the presence of certain congenital abnormalities in the patient, as well as the possibility of severe gastric reflux and/or a high rate of aspiration. In other situations, nutritional targets may not be attained at a satisfactory rate through G-tube feeding. In such patients, feeding may often be accomplished by inserting a feeding tube, sometimes referred to as a jejunostomy tube, or a "J"-tube, directly into the jejunum of the patient. The J-tube bypasses the stomach, thereby avoiding congenital abnormalities, and decreasing the risk of gastric reflux and aspiration. In addition, the J-tube often provides better success in delivering nutrients than a G-tube, and allows the nutrients to be delivered and absorbed more rapidly.

Notwithstanding the foregoing, however, there are some difficulties associated with the use of jejunostomy tubes. For example, due to the generally offset position of the jejunum relative to the stomach, it is often difficult to properly direct the distal end of a J-tube into the jejunum. J-tubes are also typically very flexible, which contributes to the difficulty in directing the tubes to the desired area. In addition, once positioned, J-tubes are subject to dislodgement.

In view of the difficulties encountered in placing such tubes in the jejunum, radiographic imaging techniques, e.g., x-ray, are generally utilized to verify proper placement of such tubes. Since health care workers must transport the patient to the radiology facility to obtain the x-ray, this technique increases the cost and complexity of the feeding tube placement. In addition, the use of radiographic imaging exposes the patient to radiation. If the x-ray indicates that an unsuitable placement was achieved, then the verification process must be repeated following another attempt at placement. This adds still more cost and complexity to the procedure, and further increases the amount of radiation to which the patient is exposed.

Ultrasound visualization is an alternative imagining modality. Ultrasound visualization has favourable characteristics in that it can be performed at the bedside, and eliminates radiation exposure to the patient. For a prior disclosure of a catheter having echogenicity enhancement reference is directed to US 5,967,988 which discloses a retrograde coronary sinus perfusion catheter which includes a flexible, tubular catheter body and an inflatable balloon. The inflatable balloon is located adjacent the distal end of the catheter body. An echogenicity enhancement is embedded within the catheter body which is adapted to reflect ultrasonic waves at a characteristic different from the catheter body. However, the use of ultrasound visualization can be problematic if a volume of air/gas is present between the ultrasound transducer head and a structure being visualized. The gastrointestinal tract has a generally "pipe-like" configuration along much of its length. As the feeding tube advances along the GI tract during insertion, it may track the posterior intestinal wall of this tract, leaving an air gap within the intestinal lumen along the anterior wall. Since the transducer head is positioned on the side of the anterior wall, the presence of the air gap inhibits optimal visualization of the feeding tube under ultrasound.

It would be desirable to provide a feeding tube suitable for placement in the jejunum of the patient, wherein the feeding tube is structured in a manner such that proper placement of the feeding tube may be verified by means readily available at the patient's bedside, and by means that do not expose the patient to harmful radiation.

### BRIEF SUMMARY

The problems of the prior art are addressed by the feeding tube disclosed herein. In one form, a tube for insertion into a body passageway of a patient is described. An elongated tubular member has a proximal portion, a distal portion, a lumen extending between the proximal and distal portions, and at least one aperture at the distal portion sized and positioned for passage of fluid material therethrough between the lumen and a target area at the body passageway external to the tubular member. An inflatable member is disposed about a length of the elongated tubular member distal portion. The inflatable member is sized and configured such that upon inflation, the inflatable member projects outwardly from the tubular member distal portion so as to be engageable with an interior wall of the body passageway. An echogenic material is disposed along the tubular member distal portion for providing an echogenic capability such that the tubular member length is visible under ultrasound visualization.

In another form, a tube for insertion into a body passageway of a patient is described. The tube comprises an elongated tubular member having a proximal portion, a distal portion, a lumen extending between the proximal and distal portions, and at least one aperture at said distal portion sized and positioned for passage of fluid material therethrough between the lumen and a target area at the body passageway external to the tubular member. An inflatable member is disposed about a length of the elongated tubular member distal portion. The inflatable member is sized and configured such that upon inflation the inflatable member projects outwardly from the tubular member distal portion so as to be engageable with an interior wall of the body passageway. The inflatable member includes an echogenic material engaged therewith in a manner such that the inflatable member is visible under ultrasound visualization.

### BRIEF DESCRIPTION OF THE DRAWINGS

Fig. 1 is a side view of the distal end of a tube having an echogenic enhancement, according to an embodiment of the present invention;
Fig. 2 is an enlarged cross-sectional view of the tube taken along line 2-2 of Fig. 1;
Fig. 3 is a side view of the proximal end of the tube of Fig. 1;
Fig. 4 is a side view of the distal end of a tube having an echogenic enhancement, according to another embodiment of the present invention;
Figs. 5 and 6 are side views of the distal end of a tube having an echogenic enhancement, according to still further embodiments of the present invention;
Fig. 7 is a cross-sectional view similar to that of Fig. 2, but wherein the inflation tube is exterior of the tube;
Fig. 8 is a side view of a portion of the interior of the jejunum, illustrating the distal end of the tube prior to inflation of the balloon, and showing the air gap present between the echogenic portion of the tube and the ultrasound transducer head;
Fig. 9 is a side view of the interior of the jejunum as in Fig. 8, wherein the balloon has been inflated in a manner to eliminate the air gap;
Fig. 10 is a side view of an alternative embodiment of a feeding tube, illustrating an echogenic enhancement applied directly to the balloon; and
Fig. 11 is a view of the feeding tube of Fig. 10, wherein the balloon is in an inflated condition.

### DESCRIPTION OF PREFERRED EMBODIMENTS

For purposes of promoting an understanding of the present invention, as defined by the claims, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" will be used to describe the opposing axial ends of the inventive feeding tube, as well as the axial ends of various component features. The term "proximal" is used in its conventional sense to refer to the end of the feeding tube (or component thereof) that is closest to the operator during use. The term "distal" is used in its conventional sense to refer to the end of the feeding tube (or component thereof) that is initially inserted into the patient, or that is closest to the patient during use.

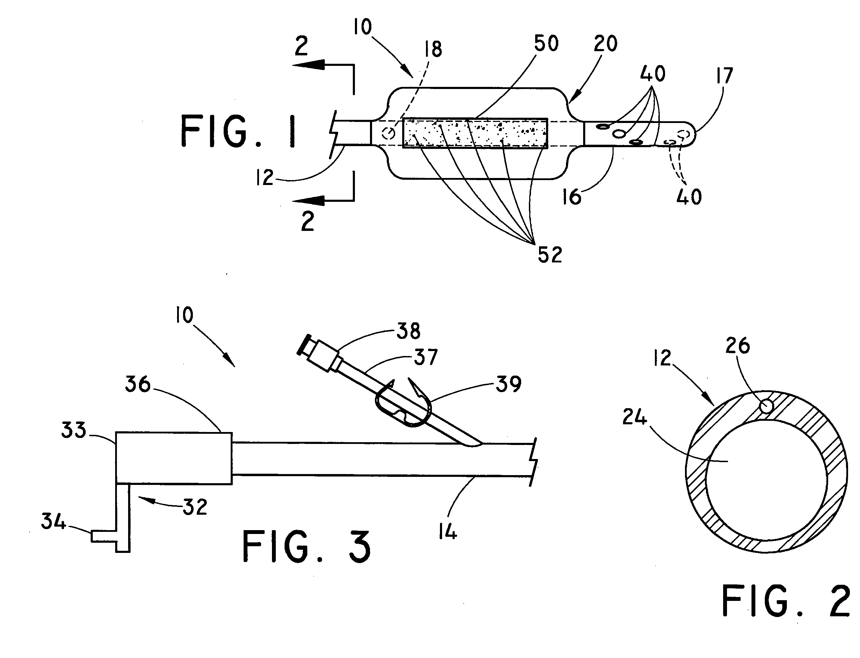

Figs. 1-3 illustrate one embodiment of a feeding tube 10 according to the present invention. Feeding tube 10 may be a jejunal feeding tube. Typically, a jejunal feeding tube is inserted into the jejunum via the oral cavity (nose or mouth). In the following non-limiting description, feeding tube 10 will be referred to as a naso-jejunal feeding tube, that is, a feeding tube inserted via the nasal cavity. Those skilled in the art will appreciate that in an appropriate case feeding tube 10 may be inserted into the jejunum other than through the nasal cavity, e.g., through the stomach or through the mouth. In addition, feeding tube 10 need not necessarily be advanced such that the distal opening extends into the jejunum. Rather, in some instances, the distal end of the tube may be positioned in other portions of the small intestine. It is generally preferred, however, that the distal end of the tube resides in the jejunum. In addition to providing nutritional products into the jejunum, tube 10 may also be used for delivering other fluid materials, such as drugs and/or contrast materials, to other locations in the alimentary canal.

Although referred to herein generally as "liquid" or "fluid" products, those skilled in the art will appreciate that the materials transported through the tube may be of various consistencies and viscosity. Therefore, it is to be understood that the "liquid" or "fluid" products referred to herein may also include some solid and/or semi-solid portions, as well known in the art.

Feeding tube 10 comprises an elongated tubular member 12 having a proximal portion 14 (Fig. 3) and a distal portion 16 (Fig. 1). An inflatable member, such as balloon 20, is disposed at distal portion 16 of the tubular member. Feeding tubes, such as naso-jejunal tubes, are well known in the art, and tubular member 12 may be formed from any compositions commonly used and/or appropriate for such purposes, such as PVC, polyurethane and silicone. Typically such tubular members have a length of about 150-160 cm, and an outer diameter of between about 8 and 16 French (2.6 to 5.3 mm). Most commonly, the length of the tubular member is about 155 cm and the outer diameter is about 14 French (4.6 mm). Those skilled in the art will appreciate that the length and diameter of a feeding tube may be varied to account for differences in patient size.

As shown in Fig. 2, two lumens 24, 26 extend through at least a portion of elongated tubular member 12. A large diameter lumen 24 is sized to enable passage therethrough of the fluid material, such as nutritional products. A small diameter lumen 26 is sized for passage therethrough of an inflation medium for use in inflating balloon 20. The preferred inflation medium is of a type that allows penetration by ultrasound signals when tubular member distal portion 16 is disposed along a body passageway. Typical inflation media, such as air and gases having a low density comparable to that of air, are not generally preferred since these media have a tendency to deflect ultrasound signals at a transition between the tissue of the body passageway, and the air/gas inflation medium. Rather, inflation media having a density greater than that of air are particularly preferred. Water and saline are non-limiting examples of such media. Other fluids having a density suitable for transmitting ultrasound signals from within a body passageway as described are also particularly preferred.

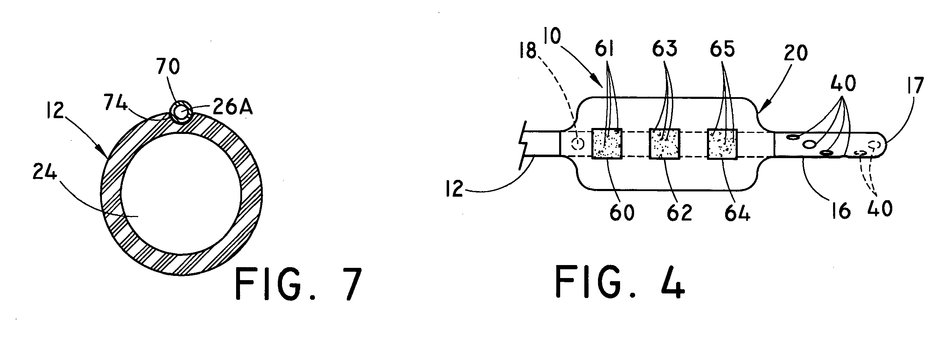

Although the feeding tube in the preferred embodiments described herein includes two lumens, the tube can be fashioned to have more, or fewer, lumens. For example, although the inflation lumen 26 in Fig. 2 is shown passing through the interior of tubular member 12, the inflation lumen may alternatively be fashioned as a tube extending along, and adhered to, the external surface of the tubular member. As shown in Fig. 7, a small diameter tube 70 extends longitudinally along the outer surface of elongated tubular member 12. Inflation lumen 26A extends through the interior of small diameter tube 70. In this case, inflation port 18 may be omitted, and the distal end of tube 70 extends directly into the interior space of balloon 20. In the embodiment shown in Fig. 7, elongated tubular member 12 is provided with a shallow longitudinal channel 74 along its outer surface. Small diameter tube 70 tracks the channel from the source for the inflation fluid to the interior of the balloon. Inflation lumen 26A extends through tube 70 and communicates with the interior space of the balloon, as in the embodiment of Fig. 2. In a variation of this embodiment, small diameter tube 70 may be glued or otherwise adhered directly to the outer surface of tubular member 12, and longitudinal channel 74 may be omitted.

Fig. 3 illustrates the proximal portion 14 of tube 10. In the embodiment shown, proximal portion 14 includes an optional adapter 32 suitable for engagement with a reservoir (not shown) of the fluid material, such as nutritional products. Adapter 32 includes an entryway 33 that is sized and shaped for communication by any well-known means with the fluid reservoir. A plug 34 may be provided for selectively opening and closing entryway 33. Adapter distal portion 36 is sized to snugly fit over tube proximal portion 14 in any conventional manner, and to establish communication between entryway 33 and large diameter lumen 24 for transmission therethrough of the fluid material. Alternatively, a conventional connector can be used to join adapter 32 and feeding tube proximal portion 14 in well-known fashion.

A luer fitting 38 or other suitable connector is provided for connection to a source (not shown) for the inflation fluid. An extension tube 37 extends from luer fitting 38 to establish communication by any conventional means with small diameter lumen 26 for transmission of the inflation fluid. A conventional valve or clamp 39, or a stop-cock, may be provided for controlling flow of the inflation fluid through extension tube 37 in well known fashion.

Proximal tube portion 14 and adapter 32 as shown and described are conventional, and further discussion of them is not germane to an understanding of the features of the present invention. Those skilled in the art will appreciate that other conventional proximal fittings, connectors, etc., used for establishing communication with the various lumens of a multi-lumen catheter may be substituted for the arrangement illustrated in Fig. 3, and are considered within the scope of the present invention.

As shown in Fig. 1, tube distal portion 16 further comprises one or more side ports, or apertures, 40 disposed along the distal portion of the tube. Apertures 40 provide openings through which nutritional products or other fluid material can exit the interior of the feeding tube, and enter the jejunum. Typically, apertures 40 are round or elliptical, and have dimensions well known in the art (e.g., between about 2.5 and 4 mm maximum diameter). Those skilled in the art will appreciate that apertures of other configurations and dimensions may be substituted. The feeding tube apertures 40 may be sequentially disposed along opposite sides of the feeding tube, e.g., at approximately 2 cm intervals. Alternatively, apertures 40 may be randomly distributed along tube distal portion 16 in any fashion that permits passage therethrough of liquid or fluid products in a manner suitable for the intended use of tube 10.

Preferably, the distal tip 17 of tube 10 is closed. When the distal tip is closed, the fluid material passes through apertures 40. In addition, with a closed distal end, a conventional guiding or tracking member (e.g., stiff wire guide, mandrel, stylet) may be used to aid initial insertion of the tube if desired. In some instances, however, it may be preferred to maintain an open distal end to permit passage of liquid product therethrough. When the feeding tube has an open distal end, the presence of apertures 40 is optional.

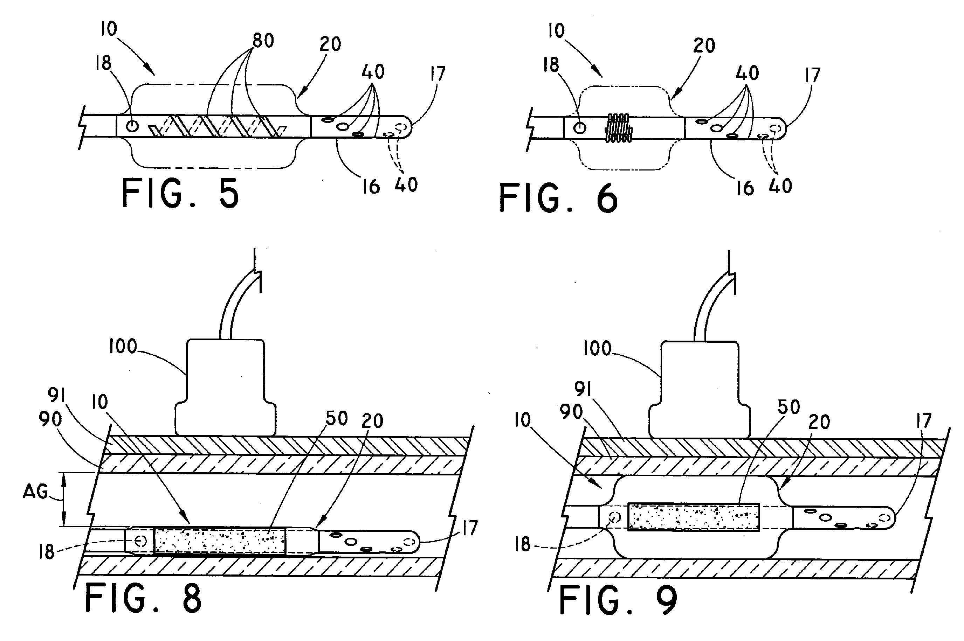

Inflatable balloon 20 is positioned along tube distal end 16. In the non-limiting embodiment of Fig. 1, balloon 20 has a generally cylindrical outer surface 23. Providing a generally cylindrical outer surface to the balloon optimizes the contact area between the balloon and the interior wall of the jejunum upon inflation of the balloon (Fig. 9). Preferably, balloon 20 is one of the category of balloons known as "compliant" balloons. Although compliant balloons are preferred for use herein, in an appropriate case, a "semi-compliant" or a "non-compliant" balloon will also be suitable.

The degree of compliance of a balloon refers to the ability of the balloon diameter to increase with increasing pressure. Generally, compliant balloons are capable of greater increase in diameter upon inflation than semi-compliant balloons, which in turn are capable of greater increase in diameter than non-compliant balloons. When a compliant balloon is utilized, a particular balloon 20 can be readily used with patients of various sizes, and having anatomic features of a variety of sizes and shapes.

Compliant balloons are preferred for use herein, as such balloons are readily capable of expansion until the outer surface of the balloon contacts the interior jejunum wall. Typically, such balloons are made from materials such as silicone, urethane, latex, or a silicone/polyurethane copolymer. Non-compliant and semi-compliant balloons, on the other hand, are typically made from materials such as polyethylene terephthalate (PET), polyamides (nylons), or a polyether block amide, such as PEBAX®. These balloons remain substantially at a pre-selected diameter as the internal balloon pressure increases beyond that required to fully inflate the balloon. The use of compliant, semi-compliant, and non-compliant balloons is now well known in the medical arts, and those skilled in the art can readily select an appropriate balloon composition for a particular application.

The respective proximal 21 and distal 22 ends of the balloon are secured to the tubular member 12 by conventional techniques, such as via heat bonding. From the standpoint of ease of bonding, it is often preferred to utilize a balloon formed of the same or a similar material as the elongated tubular member. However, when the balloon and the tubular member are formed of different materials, secure connections can nonetheless be achieved by use of suitable adhesives known for such use in the medical field, such as cyanoacrylates. Those skilled in the art are well aware of suitable techniques for securing a balloon to a tubular member for medical applications, and other well-known techniques may be substituted for those described.

Distal portion 16 of elongated tubular member 12 includes at least one inflation port 18 sized and positioned for transmission of the inflation medium via inflation lumen 26 to the interior space of balloon 20. Preferably, balloon 20 is positioned proximal of apertures 40 along the length of elongated tubular member 12 as shown, to prevent interference with the flow of the nutritional products or other fluid from large diameter lumen 24 through the apertures.

Those skilled in the art will appreciate that the feeding tube may include additional features well known in the art. For example, the outer surface of the elongated tubular member may be provided with a series of projections, such as fins or fin-like elements, along the distal portion thereof to enhance the advancement of the feeding tube into the jejunum via peristalsis. Feeding tubes having features that promote self-advancement by peristalsis are further described in, for example, U.S. Patent Nos. 6,589,213 and 6,767,339. Additionally, the feeding tube may be provided with a series of markings displayed at discrete locations along the length of the tubular member to enable the health care worker to monitor the advancement of the tube into the jejunum.

As stated above, the elongated tubular member can be provided with one or more additional lumens that extend through at least a portion of the length of the tubular member. Such additional lumens could be used, e.g., to provide an additional fluid source, such as providing a liquid medication in addition to the liquid nutritional products, and/or to provide a conduit through which various pressures or functions within the patient's body may be monitored. An increase in the number of lumens may necessitate minor alteration in the features of the inventive tube as described herein, such as the location of the apertures at the distal end of the tubular member; however those skilled in the art can readily make such alterations when following the teachings of the present invention.

It is sometimes difficult to direct the distal tip of a tube to a target site within the anatomy of a patient that is off-set, or has an otherwise non-direct path, relative to an entry site. A feeding tube that is intended to be directed into the jejunum of a patient is an example of such a tube. Due to the difficulty in inserting tubes to such sited, it is generally desirable to verify the proper placement of the tube following insertion. When verifying the placement of a feeding tube in the jejunum, the normal procedure is to transport the patient to the radiology facility to obtain an x-ray. However, this procedure increases the cost and complexity of the feeding tube placement. In addition, the procedure exposes the patient to radiation.

The feeding tube described herein includes an echogenic capability that enables the health care worker to observe in real time the location of the end of a tube in a body passageway, such as the jejunum. The presence of the echogenic capability enables tip confirmation to be carried out under ultrasound visualization at the patient's bedside. As a result, there is no need to transport the patient to another location for further verification, and the patient need not be exposed to harmful radiation.

In the embodiment shown in Fig. 1, a reflective surface capable of visualization under ultrasound is provided along the length of the elongated tubular member. In this embodiment, the reflective surface comprises an echogenic sheath 50 disposed along the tubular member distal length interiorly of the balloon. Sheath 50 may comprise a conventional tube or sleeve that covers all or a part of the length of the tubular member that is disposed interior of the balloon. The sheath is preferably formed of a metal or metal alloy, and is provided with a surface capable of scattering and/or reflecting ultrasound energy back to the ultrasound head in a manner that enhances the echogenic capacity of the sheath.

In order to apply sheath 50 to the tubular member 12, the tubular member 12 may be stretched in longitudinal fashion to a smaller diameter, and the sheath may be inserted thereover. When the stretching is relaxed, tubular member 12 returns to its original diameter, and sheath 50 is snugly engaged therewith. Alternatively, a sheath 50 may be placed within the lumen of the tubular member, and the sheath is locked in place as the tubular member distal end 17 is closed in a conventional heated tip-forming device. As another alternative, an adhesive can be applied to the tubular member, and the sheath can be affixed to the tubular member via the adhesive. It is known to fashion echogenic bands over tubular members, such as catheters, and a skilled artisan can readily fashion a suitable technique herein. Tubular members having echogenic bands or sheaths applied thereto are known in the art for other purposes. Examples of catheters include the ECHOTIP® ureteral catheter, and the ECHOTIP® Soft-Pass Embryo Transfer catheter, both available from Cook Medical, of Bloomington, IN.

In the embodiment shown, the echogenic surface comprises a series of irregularities, such as deformations 52, distributed along the exterior surface of sheath 50. Deformations 52 are imperfections that are formed along the surface of the sheath in a manner that enhances the ability of the sheath to scatter and/or reflect the ultrasound energy. The deformations may be formed along the length of the sheath by well-known processes, such as media blasting, physical deformation, machining (e.g., knurling on a lathe), micro-dimpling, etc. Those skilled in the art will appreciate that there are many other ways of forming deformations in a substrate of a type that will result in the scatter and/or reflectance of ultrasound signals, and that may be substituted for the techniques described above. Deformations 52 should be formed in a manner such that they do not adversely affect the mechanical properties of the sheath in any material fashion.

The presence of the imperfections, such as deformations 52, causes ultrasound waves that contact the deformations to travel in multiple directions and in generally random fashion. The increase in scatter and/or reflection of the ultrasound waves enhances the temporal visualization of the tip of the feeding tube during ultrasound examination. By viewing the ultrasound signals created thereby, the health care worker may confirm proper tip location. Alternatively, if it is determined that the desired tip location has not been achieved, the location of the tube may be adjusted on-site.

Although the echogenic surface has been described hereinabove as a sheath that is positioned over the portion of the tubular member, other means for enhancing echogenic signals may be substituted. For example, rather than providing a sheath 50 as described, in an alternative embodiment, one or more echogenic cannulae 60, 62, 64 may be provided, as shown in Fig. 4. Cannulae 60, 62, 64 may comprise irregularities, such as a plurality of deformations 61, 63, 65, distributed along the surfaces of the respective cannulae. When present, the cannula(e) may be applied to the sheath in the same or a similar manner as sheath 50. Utilizing one or more cannulae in place of an elongated sheath may enhance the flexibility of the echogenic portion of the feeding tube when compared to use of the sheath.

Another embodiment illustrating echogenic enhancement of a tubular member is shown in Fig. 5. In this embodiment, rather than providing a sheath or cannula(e) as described above, an echogenic coiled ribbon 80 is wrapped around all, or a portion, of the length of tubular member 12 interior of balloon 20. Ribbon 80 may be formed of the same or a similar composition as sheath 50, such as a metal or metal alloy, and deformations are disposed along the surface of the ribbon. The deformations may be formed in the same manner as the deformations on the structures previously described, and may be randomly dispersed along the surface of ribbon 80. The deformations may be applied to ribbon 80 before coiling of the ribbon, or after coiling with the aid of a mandrel or other reinforcement.

Echogenic ribbon 80 may be fitted over distal portion 16 of tubular member 12 by any convenient mechanism, such as wrapping it around the distal portion, or by stretching the distal end of tubular member, and applying the ribbon to the stretched end. If desired, an adhesive can be utilized to provide a more secure attachment. As another alternative, ribbon 80 can be embedded into the wall of the tubular member, and a plastic can be extruded or otherwise applied over the ribbon. Utilizing a coiled ribbon 80 as described herein provides added flexibility to the distal end of the tube when compared to the use of the elongated sheath, which may facilitate passage into the jejunum.

Although ribbon 80 is shown in Fig. 5 as a continuous ribbon helically wrapped around the tubular member, other configurations are possible. For example, if desired, multiple shorter ribbons can be wrapped around the tubular member 12 interior of balloon 20, in the same manner as the use of multiple cannulae 60, 62, 64 in the embodiment of Fig. 4. This arrangement may also provide additional flexibility to the echogenic portion of the feeding tube.

Another variation is shown in Fig. 6. In this embodiment, rather than providing a ribbon having deformations formed along the surface of the ribbon, a small echogenic wire, e.g., a round wire formed of a metal or metal alloy such as stainless steel, may simply be wound around the tubular member distal end 16. In this case, the surface of the wire acts in the nature of the deformations, as the density of the wire relative to that of the tubular member imparts an echogenicity to the wire.

As yet another alternative, instead of utilizing a separate echogenic sheath, ribbon, etc., the tubular member may be formed to have an echogenic material incorporated into all or any designated portion of its matrix. One example of this is to incorporate a known echogenic material, such as glass spheres, echogenic metal or alloys (e.g., tungsten), etc., into the extrusion composition for the tubular member. Glass spheres, as well as various metals and alloys, are known to enhance echogenicity, and in this case, these materials may be readily incorporated into the polymer matrix for the feeding tube. Preferably, however, the materials (e.g., the glass spheres) will only be incorporated into the distal portion of the tubular member. In this event, the distal portion of the tubular member having the echogenic enhancement can be affixed (e.g., via heat bonding, adhesion, etc.) to the proximal portion. Once this tubular member is formed, there would be no further need to add other materials to enhance echogenicity, as sufficient echogenicity for visualization under ultrasound is provided by the matrix materials. The balloon would then be affixed to the tube in the same manner as before.

Those skilled in the art will appreciate that other known ways of imparting echogenicity to a material may be substituted for those described herein, including, e.g., the techniques disclosed in U.S. Patent Application Publ. 2008/0097213.

The following example describes one preferred use of the inventive tube, namely as a naso-jejunal feeding tube for transmitting nutritional products from the nasal cavity to the jejunum. Those skilled in the art will appreciate that with minor modification, the tube may be inserted other than through the nose, and may be advanced to destinations other than the jejunum.

Initially, the distal end of the tube is inserted through the oral cavity, and is advanced into the stomach with the balloon in an uninflated condition. A conventional guiding or tracking member (e.g., stiff wire guide, mandrel, stylet) can be used if desired to assist in advancing the tube into the stomach. If the guiding or tracking member is used, it is removed once advancement into the stomach is achieved. Insufflation and auscultation may be used to confirm that the distal tip of the feeding tube is in the stomach.

The feeding tube is then advanced through the stomach of the patient such that the distal portion of the generally elongated tubular member extends into the small intestine of the patient. Preferably, the distal end portion 16 of the tubular member is advanced into the small intestine via peristaltic activity along the GI tract. In cases of weak peristaltic activity, pharmacological agents may be used to increase this activity pursuant to well-known techniques. Reliance on peristaltic activity is optional, however, and other known means of directing a feeding tube to the jejunum may be employed, either in conjunction with peristalsis, or as an alternative to peristaltic activity.

Once the distal end of the feeding tube is believed to have reached the jejunum, the next step is to confirm placement of the tip in the jejunum. In many cases, the feeding tube advances along the posterior wall of the small intestine upon insertion. An example of this arrangement is shown in Fig. 8. The transducer head 100 is positioned along an anterior body wall 91 in the vicinity of the jejunum 90. As shown, there is an air gap AG present in the jejunum between the tube and the transducer head. The presence of an air gap between the transducer head and the echogenic target adversely affects the strength of an ultrasound signal. This gap, comprising a low density fluid such as air and/or other body gases, may cause a deflection of the ultrasound signal such that it does not penetrate to the echogenic surface. Therefore, to achieve optimal visualization, the presence of the air gap between the tube and the transducer head should be minimized, if not eliminated altogether.

As shown in Fig. 8, the balloon is in an uninflated condition. In order to inflate the balloon, clamp 39 (Fig. 3) is released, and the inflation medium is transmitted via extension tube 37, inflation lumen 26, and inflation port 18 to the interior of balloon 20. Sufficient medium is introduced to inflate the balloon, such that generally cylindrical outer balloon surface 23 is flush with the interior wall of the jejunum 90. This is shown in Fig. 9. Following inflation of the balloon, clamp 39 is re-tightened along extension tube 37 to maintain the balloon in the inflated condition. If desired, the volume of fluid within balloon 20 may be monitored in conventional fashion (e.g., utilizing a pilot balloon).

As stated above, the medium selected for use herein as the inflation medium is of a type that allows suitable penetration by ultrasound signals. Air and/or gases having a density similar to that of air, will not typically be suitable inflation media, as they do not have sufficient density to adequately transmit ultrasound signals. On the other hand, media having a greater density than air, such as water, saline, and other fluids having a density similar to, or greater than, that of water and/or soft body tissue are particularly suitable. Those skilled in the art are capable of selecting an appropriate inflation fluid based upon the discussion provided herein.

Upon inflation of the balloon as shown in Fig. 9, the air gap AG between the echogenic portion of the tube and the anterior jejunal wall is at least substantially eliminated, thereby permitting penetration by the ultrasound signals to the echogenic surface. By utilizing an inflation medium that permits penetration by ultrasound, the health care worker may determine the position of the feeding tube distal end in real time by conventional ultrasound examination. The ultrasound head 100 typically includes a series of piezoelectric effect crystals in alignment. The ultrasound head is capable of sending out a series of ultrasound beams, and receiving reflected energy. The reflected energy is amplified, processed, and integrated, all in well known fashion, to provide a real-time image on the monitor.

If suitable placement is confirmed by the ultrasound image, the balloon may be deflated by once again releasing clamp 39, and withdrawing the medium (e.g., via a syringe), or otherwise allowing the medium to drain from the interior of the balloon. Following deflation of the balloon, the clamp is re-set, and the feeding tube may be secured to the patient in any conventional fashion. The adapter 32 at the proximal end of the feeding tube is then engaged by conventional means to the fluid reservoir, and the nutritional products are transmitted through the feeding tube into the jejunum of the patient.

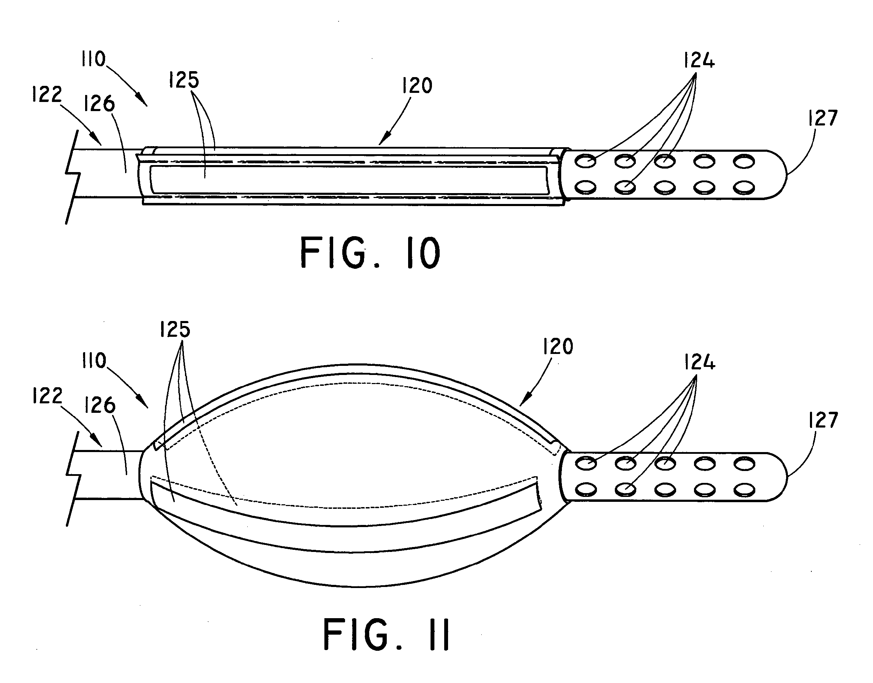

Figs.10-11 illustrate another embodiment of a feeding tube 110. In this embodiment, an echogenic material is provided along the body of an inflatable member, such as a balloon. The echogenic material can be provided along the balloon in a variety of ways. One particularly preferred manner of imparting echogenicity to a balloon is to adhere or otherwise apply echogenic materials/strips to the surface of the balloon. Echogenic strips/materials can be of any composition and configuration capable of imparting suitable echogenicity for visualization under ultrasound imaging techniques, such as the compositions and configuration described hereinabove. The echogenic strips/materials can be provided with deformities as also described above. If desired, the uninflated balloon can be pleated or folded in a manner that is similar to known balloon folding techniques utilized in connection with, e.g., atherectomy balloons having cutting materials applied to the outer surface of the balloon, such as illustrated in U.S. Pat. Publ. No. 2006/0149308.

Fig. 10 illustrates one non-limiting manner in which one or more echogenic members, such as echogenic strips 125, may be applied to a balloon 120 of feeding tube 110. Tubular member 122 includes a distal portion 126, having a distal tip 127, and a plurality of apertures 124. As stated above, apertures 124 may be provided in a variety of configurations and orientations along the distal portion of the balloon. The arrangement in Figs. 10 and 11 illustrates another non-limiting example of a suitable orientation of apertures 124. As illustrated, one or more echogenic strips 125 can be adhered or otherwise applied to the outer surface of the balloon. In the embodiment shown, the echogenic strips 125 are applied longitudinally along the length of the balloon with a suitable adhesive. Any number of echogenic strips may be applied along, or around, the circumference of balloon, in any desired orientation.

Upon transmission of an inflation medium as discussed above, balloon 120 is inflated to a suitable configuration, such as that shown in Fig. 11. Echogenic strips 125 are visible under ultrasound in the manner discussed above, whereby suitable placement of the distal end of the tube in the jejunum may be verified. As shown in the non-limiting embodiment of Fig. 11, three strips 125 are longitudinally spaced at approximately 120 degree intervals, along the length of the balloon. Although any number of strips may be provided, by providing at least three strips 125 as described, balloon 120 will generally be readily visible under ultraviolet visualization at any given orientation, or rotation, of the balloon in the jejunum.

Although balloon 120 shown in Figs. 10 and 11 has been echogenically enhanced by use of longitudinal strips 125 as shown, those skilled in the art will appreciate that other types of echogenic enhancement may be incorporated into, or onto, the balloon. For example, rather than longitudinal strips, the echogenic features may comprise echogenic patches, rings, bands, or like features distributed in various ways along the interior or exterior surface of the balloon. As a further alternative, the balloon itself can be formed to include echogenic materials or particles, such as glass spheres, provided in, or on, the matrix of the balloon. As a still further alternative, an echogenic coating can be applied to the balloon. Suitable echogenic coatings are described in, for example, U.S. Patent Nos. 6,506,156 and 6,106,473.

While these features have been disclosed in connection with the illustrated preferred embodiments, other embodiments of the invention will be apparent to those skilled in the art that come within the scope of the invention as defined in the following claims.

## Claims

1. A feeding tube (10) for insertion into a body passageway of a patient, comprising:
an elongated tubular member (12) having a proximal portion (14), a distal portion (16), a lumen (24) extending between said proximal and distal portions, and at least one aperture (40) at said distal portion, said aperture sized and positioned for passage of fluid material therethrough between said lumen and a target area at said body passageway external to said tubular member;
an inflatable member (20) disposed about a length of said elongated tubular member distal portion, said inflatable member sized and configured such that upon inflation said inflatable member projects outwardly from said tubular member distal portion so as to be engageable with an interior wall of the body passageway; and
an echogenic material (50, 52, 60-65, 80, 125) disposed along said tubular member distal portion or engaged with said inflatable member, so that in a working configuration with the inflatable member uninflated an air gap exists between the echogenic material and the interior wall of the passageway preventing visualization of the echogenic material under ultrasound imagery and that in a placement verification configuration the inflatable member is inflated to eliminate said air gap and render the echogenic material visible under ultrasound imagery.

2. The tube of claim 1, wherein said echogenic material is disposed interiorly of said inflatable member along said tubular member distal portion.

3. The tube of claim 1 or claim 2, wherein said lumen comprises a first lumen, said elongated tubular member further comprising a second lumen, said second lumen communicating with an interior space of said inflatable member for transmission of an inflation fluid into said interior space, said inflation fluid capable of penetration by ultrasound signals when said inflatable member is received in said body passageway.

4. The tube of any one of the preceding claims, wherein upon inflation said inflatable member comprises a generally cylindrical outer surface and preferably
wherein said inflatable member comprises a compliant balloon inflatable to said generally cylindrical outer configuration.

5. The tube of any one of the preceding claims, wherein the inflatable member is positioned proximal of said at least one aperture along said elongated tubular member length.

6. The tube of any one of the preceding claims, wherein said echogenic material comprises a metal or metal alloy engaged with said tubular member distal portion, said metal or metal alloy having deformations distributed along an exterior surface thereof.

7. The tube of any one of the preceding claims, wherein said echogenic material comprises a band disposed around said tubular member distal portion.

8. The tube of any one of the preceding claims, wherein said echogenic material is incorporated into a matrix of said tubular member distal portion disposed interiorly of said inflatable member.

9. The tube of claim 1, wherein said echogenic material is applied to a surface of said inflatable member.

10. The tube of claim 9, wherein said echogenic material comprises deformities distributed along a surface thereof for enhancing an echogenicity of said inflatable member.

11. The tube of claim 9, wherein said echogenic material comprises one or more echogenic strips applied to said surface.

12. The tube of claim 1, wherein said echogenic material comprises an echogenic coating engaged with a surface of said inflatable member.

13. The tube of claim 1, wherein said echogenic material is incorporated into a matrix of said inflatable member.

14. The tube of any one of the preceding claims configured for insertion into the jejunum of a human patient for delivery of nutrient material.

## Patentansprüche

1. Ernährungssonde (10) zum Einführen in einen Körperdurchgang eines Patienten, umfassend:
ein längliches röhrenförmiges Element (12) mit einem proximalen Abschnitt (14), einem distalen Abschnitt (16), einem Lumen (24), das sich zwischen dem proximalen und dem distalen Abschnitt erstreckt, und mindestens einer Öffnung (40) am distalen Abschnitt, die so bemessen und positioniert ist, dass Fluidmaterial dort zwischen dem Lumen und einem Zielbereich an dem Körperdurchgang, der außerhalb des röhrenförmigen Elements liegt, hindurchgehen kann,
ein aufblasbares Element (20), das um eine Länge des distalen Abschnitts des länglichen röhrenförmigen Elements angeordnet ist und so bemessen und konfiguriert ist, dass das aufblasbare Element beim Aufblasen vom distalen Abschnitt des röhrenförmigen Elements nach außen vorragt, damit es mit einer Innenwand des Körperdurchgangs in Eingriff gebracht werden kann, und ein echogenes Material (50, 52, 60 - 65, 80, 125), das entlang dem distalen Abschnitt des röhrenförmigen Elements angeordnet ist oder mit dem aufblasbaren Element in Eingriff steht, so dass in einer Arbeitskonfiguration bei nicht aufgeblasenem aufblasbarem Element zwischen dem echogenen Material und der Innenwand des Durchgangs ein Luftspalt vorliegt, der eine Visualisierung des echogenen Materials bei der Ultraschall-Bildgebung verhindert, und in einer Platzierungsverifizierungskonfiguration das aufblasbare Element aufgeblasen ist, um den Luftspalt zu eliminieren und das echogene Material bei der Ultraschall-Bildgebung sichtbar zu machen.

2. Sonde nach Anspruch 1, wobei das echogene Material innerhalb des aufblasbaren Elements entlang dem distalen Abschnitt des röhrenförmigen Elements angeordnet ist.

3. Sonde nach Anspruch 1 oder 2, wobei das Lumen ein erstes Lumen umfasst, wobei das längliche röhrenförmige Element ferner ein zweites Lumen umfasst, das mit einem Innenraum des aufblasbaren Elements zur Übertragung eines Aufblasfluids in den Innenraum in Verbindung steht, wobei das Aufblasfluid von Ultraschallsignalen durchdrungen werden kann, wenn das aufblasbare Element in dem Körperdurchgang aufgenommen ist.

4. Sonde nach einem der vorhergehenden Ansprüche, wobei das aufblasbare Element beim Aufblasen eine allgemein zylindrische Außenfläche umfasst und
vorzugsweise
wobei das aufblasbare Element einen nachgiebigen Ballon umfasst, der zu der allgemein zylindrischen äußeren Konfiguration aufgeblasen werden kann.

5. Sonde nach einem der vorhergehenden Ansprüche, wobei das aufblasbare Element proximal von der mindestens einen Öffnung entlang der Länge des länglichen röhrenförmigen Elements positioniert ist.

6. Sonde nach einem der vorhergehenden Ansprüche, wobei das echogene Material ein Metall oder eine Metalllegierung umfasst, das bzw. die mit dem distalen Abschnitt des röhrenförmigen Elements in Eingriff steht, wobei das Metall oder die Metalllegierung Deformierungen haben, die entlang einer Außenfläche davon verteilt sind.

7. Sonde nach einem der vorhergehenden Ansprüche, wobei das echogene Material ein Band umfasst, das um den distalen Abschnitt des röhrenförmigen Elements herum angeordnet ist.

8. Sonde nach einem der vorhergehenden Ansprüche, wobei das echogene Material in eine Matrix des distalen Abschnitts des röhrenförmigen Elements eingegliedert ist, die innerhalb des aufblasbaren Elements angeordnet ist.

9. Sonde nach Anspruch 1, wobei das echogene Material auf eine Fläche des aufblasbaren Elements aufgebracht ist.

10. Sonde nach Anspruch 9, wobei das echogene Material Deformationen umfasst, die entlang einer Fläche davon verteilt sind, um die Echogenität des aufblasbaren Elements zu verbessern.

11. Sonde nach Anspruch 9, wobei das echogene Material einen oder mehrere echogene Streifen umfasst, die auf die Fläche aufgebracht sind.

12. Sonde nach Anspruch 1, wobei das echogene Material eine echogene Beschichtung umfasst, die mit einer Fläche des aufblasbaren Elements in Eingriff steht.

13. Sonde nach Anspruch 1, wobei das echogene Material in eine Matrix des aufblasbaren Elements eingegliedert ist.

14. Sonde nach einem der vorhergehenden Ansprüche zum Einführen in das Jejunum eines menschlichen Patienten zur Abgabe von Nahrungsmaterial.

## Revendications

1. Tube d'alimentation (10) destiné à être inséré dans un passage corporel d'un patient, comprenant :
un organe tubulaire allongé (12) comportant une partie proximale (14), une partie distale (16), une lumière (24) s'étendant entre lesdites parties proximale et distale, et au moins une ouverture (40) au niveau de ladite partie distale, ladite ouverture étant dimensionnée et positionnée en vue du passage d'une substance fluide à travers elle entre ladite lumière et une zone cible au niveau dudit passage corporel à l'extérieur dudit organe tubulaire ;
un organe gonflable (20) disposé autour d'une longueur de ladite partie distale de l'organe tubulaire allongé, ledit organe gonflable étant dimensionné et configuré de telle sorte que, lors du gonflage, ledit organe gonflable se renfle vers l'extérieur à partir de ladite partie distale de l'organe tubulaire de façon à pouvoir venir en contact avec une paroi intérieure du passage corporel ; et
un matériau échogène (50, 52, 60-65, 80, 125) disposé le long de ladite partie distale de l'organe tubulaire ou en contact avec ledit organe gonflable, de telle manière que, dans une configuration fonctionnelle avec l'organe gonflable non gonflé, un intervalle d'air soit présent entre le matériau échogène et la paroi intérieure du passage empêchant la visualisation du matériau échogène par imagerie ultrasonore et que, dans une configuration de vérification de positionnement, l'organe gonflable soit gonflé de façon à éliminer ledit intervalle d'air et à rendre le matériau échogène visible par imagerie ultrasonore.

2. Tube selon la revendication 1, dans lequel ledit matériau échogène est disposé à l'intérieur dudit organe gonflable le long de ladite partie distale de l'organe tubulaire.

3. Tube selon la revendication 1 ou la revendication 2, dans lequel ladite lumière comprend une première lumière, ledit organe tubulaire allongé comprenant en outre une seconde lumière, ladite seconde lumière communiquant avec un espace intérieur dudit organe gonflable en vue du transfert d'un fluide de gonflage dans ledit espace intérieur, ledit fluide de gonflage étant apte à être pénétré par des signaux ultrasonores lorsque ledit organe gonflable est reçu dans ledit passage corporel.

4. Tube selon l'une quelconque des revendications précédentes, dans lequel, lors du gonflage, ledit organe gonflable comprend une surface extérieure globalement cylindrique et de préférence dans lequel ledit organe gonflable comprend un ballonnet souple pouvant être gonflé de façon à obtenir ladite configuration extérieure globalement cylindrique.

5. Tube selon l'une quelconque des revendications précédentes, dans lequel l'organe gonflable est positionné à proximité de ladite au moins une ouverture sur la longueur dudit organe tubulaire allongé.

6. Tube selon l'une quelconque des revendications précédentes, dans lequel ledit matériau échogène comprend un métal ou un alliage métallique en contact avec ladite partie distale de l'organe tubulaire, ledit métal ou alliage métallique présentant des déformations réparties le long d'une surface extérieure de celui-ci.

7. Tube selon l'une quelconque des revendications précédentes, dans lequel ledit matériau échogène comprend une bande disposée autour de ladite partie distale de l'organe tubulaire.

8. Tube selon l'une quelconque des revendications précédentes, dans lequel ledit matériau échogène est incorporé dans une matrice de ladite partie distale de l'organe tubulaire disposée à l'intérieur dudit organe gonflable.

9. Tube selon la revendication 1, dans lequel ledit matériau échogène est appliqué à une surface dudit organe gonflable.

10. Tube selon la revendication 9, dans lequel ledit matériau échogène comprend des difformités réparties le long d'une surface de celui-ci et destinées à accroître l'échogénicité dudit organe gonflable.

11. Tube selon la revendication 9, dans lequel ledit matériau échogène comprend une ou plusieurs bandelettes échogènes appliquées à ladite surface.

12. Tube selon la revendication 1, dans lequel ledit matériau échogène comprend un revêtement échogène en contact avec une surface dudit organe gonflable.

13. Tube selon la revendication 1, dans lequel ledit matériau échogène est incorporé dans une matrice dudit organe gonflable.

14. Tube selon l'une quelconque des revendications précédentes, configuré pour être inséré dans le jéjunum d'un patient humain en vue de l'administration d'une substance nutritive.
